# EUROPÄISCHE PATENTSCHRIFT

(11) **EP 1 378 215 B1**
(45) Veröffentlichungstag und Bekanntmachung des Hinweises auf die Patenterteilung: **19.07.2006**
(21) Anmeldenummer: 03013011.6
(22) Anmeldetag: 10.06.2003
(51) Int. Cl.: A61F 2/28

(54) **Subkutanes, intramuskuläres Lager für ein starres transkutanes Implantat**
Subcutaneous, intra-muscular coupling for a rigid transcutaneous implant
Logement intramusculaire sous-cutane pour implant transcutane fixe

(30) Priorität: 01.07.2002 DE 10230074
(43) Veröffentlichungstag der Anmeldung: 07.01.2004
(73) Patentinhaber: ESKA Implants GmbH & Co., 23556 Lübeck (DE)
(72) Erfinder: Grundei, Hans, Dr. Ing., 23558 Lübeck (DE)
(74) Vertreter: Fuchs Mehler Weiss & Fritzsche

(56) Entgegenhaltungen:
- DE-A- 10 040 590
- DE-C- 19 931 882
- US-A- 4 158 895

## Beschreibung

Die Erfindung betrifft ein subkutanes, intramuskuläres Lager für ein starres transkutanes Implantat, welches intrakorporal in einem Knochenstumpf verankerbar ist und welches ein Zwischenstück zwischen dem intrakorporalen zu verankernden Teil und einer daran ankoppelbaren extrakorporalen Kopplungseinrichtung aufweist.

Ein derartiges Lager ist bekannt aus der DE-A-100 40 590. Das darin beschriebene Lager besteht aus einem flexiblen Material und es weist eine Tülle auf, die das Implantat distal fest umschließt, sowie eine intralkorporal anzuordnende Überwurfhülse in Form eines flexiblen Faltenbalges, der proximal mit einem angeformten Bund in abdichtender Weise mit der Tülle verbunden ist, derart, dass zwischen der Innenwandung und des Faltenbalges und Aussenwandung der Tülle ein Hohlraum einer Mindestbreite freibleibt. Dabei ist distal am Faltenbalg ein flexibles Gitternetzwerk angeordnet, dem sich distalseitig ein weiteres Gitternetzwerk mit einem höheren E-Modul anschließt.

Mit diesem Lager wird das Ziel verfolgt, dass sich Weichteile gegenüber dem starren Implantat bewegen können, ohne dass die Durchbruchsstelle im Körperstumpfteil einem erhöhten Risiko einer Entzündung ausgesetzt wird.

Wenn dieses bekannte Lager auch bereits in der Praxis erfolgreich eingesetzt worden ist, birgt es das Risiko, dass im Falle beispielsweise einer Reinigung der Durchtrittsstelle des Implantates durch den Oberschenkelstumpf mit einer Kanüle durch das flexible Material, in den meisten Fällen Silikon, hindurchgestochen wird und eine Verkeimung stattfindet. Dieser Möglichkeit gilt es Einhalt zu gebieten.

Allerdings vermag auch ein transkutanes Lager für ein starres Implantat gemäß der DE-C-199 31 882 dies nicht. Wie das eingangs erwähnte transkutane Lager, ist das bekannte Lager intrakorporal in einem Knochenstumpf zu verankern und weist ein Zwischenstück zwischen dem intrakorporal verankerten Teil und einer daran ankoppelbaren extrakorporalen Kopplungseinrichtung auf. Das transkutane Lager aus flexiblem Material weist eine Überwurfhülse auf, die über eine Tülle mit dem Zwischenstück verbunden ist und die in Richtung intrakorporal verschlossen und in Richtung extrakorporal offen ist.

Eine Lösung des Problems vermag auch eine Vorrichtung zum Befestigen einer Protese in einem Knochen eines Gliedmaßenstumpfes gemäß dem US-A-3,947,897 nicht anzubieten. Diese weist einen im Knochenkanal zu verankernden Sockel auf, der an der Durchtrittsstelle der Haut von einer Hülse eingefasst ist.

Vor diesem Hintergrund ist es nun die Aufgabe der vorliegenden Erfindung, ein gattungsgemäßes, subkutanes, intramuskuläres Lager der eingangs genannten Art so weiter zu bilden, dass die Sicherheit gegen eine Verkeimung der Durchtrittsstelle des Implantates und der angrenzenden Bereiche des Oberschenkelstumpfes deutlich erhöht wird.

Gelöst wird diese Aufgabe durch das Lager mit den Merkmalen des Anspruchs 1, eine Alternativelösung ist in Anspruch 2 angegeben. Vorteilhafte Weiterbildungen ergeben sich aus den Unteransprüchen.

Demgemäß wird vorgeschlagen, dass das Lager eine mit dem Zwischenstück fest verbundene starre Überwurfhülse, die in Richtung intrakorporal verschlossen und in Richtung extrakorporal offen ist, sowie eine Klemmhülse aufweist, die in die Überwurfhülse in deren offenes Ende setzbar ist. Dabei ist die Klemmhülse von einer metallischen Wolle umgeben und die Dimensionen sind so gewählt, dass die Klemmhülse in die Überwurfhülse nur unter Kompression der metallischen Wolle setzbar ist, und zwar so, dass die Kompression für einen dauerhaften Verbleib der Klemmhülse in der Überwurfhülse sorgt.

Gegenüber dem bekannten Lager ist die Überwurfhülse vorliegend ein starres Element, das nicht etwa durch Injektionskanülen durchdrungen werden kann. Zur Abdichtung des Hülseninneren wird die Überwurfhülse durch die Klemmhülse verschlossen. Letzteres freilich so, dass eine extrakorporale Kopplungseinrichtung an das Zwischenstück angekoppelt werden kann. Der Sitz der Klemmhülse in der Überwurfhülse wird durch die Kompression und Verklemmung der metallischen Wolle, die am äußeren Ende der Klemmhülse vorgesehen ist, erreicht. Die im Einsatz herrschenden Belastungskräfte bei Gebrauch der Beinprothese sorgen darüber hinaus dafür, dass die Verbindung dauerhaft bleibt.

Die feste Verbindung der Überwurfhülse an dem Zwischenstück führt dazu, dass das Implantat mit dem Lager eine starre Einheit bildet. Dies führt zu einer besonders intensiven Osteoperzeption.

Sollte letztere weniger im Mittelpunkt der Betrachtung sein, ist alternativ zu der vorstehend angegebenen Lösung eine Alternative vorgesehen. Diese unterscheidet sich von der ersten Lösung dadurch, dass die starre Überwurfhülse nicht mehr fest mit dem Zwischenstück verbunden ist, sondern an diesem elastisch festgelegt ist. Auf diese Art und Weise werden Mikrobewegungen zugelassen.

In beiden Fällen ist gemäß einer vorteilhaften Weiterbildung vorgesehen, dass die Überwurfhülse außen mit einer metallischen Wolle belegt ist. In diese metallische Wolle sprießt nach der Operation im Laufe der Zeit Haut und anderes Gewebe ein und bietet somit eine natürliche Schranke gegen eine Verkeimung.

Letztgenannte Schranke wird noch dadurch erhöht, dass die metallische Wolle, welche die Klemmhülse umgibt, sich distalseitig fortsetzt und einen umlaufenden Flansch bildet. Dieser ist gewissermaßen konzentrisch zur Hülse angeordnet.

Besonders bevorzugt wird eine Ausführungsform für beide alternativen Lösungen, wonach das Zwischenstück ein Doppelkonus mit einem zylindrischen Mittelabschnitt ist. Dieses ist per se aus der Druckschrift DE-A-100 40 590 bekannt. Diese Ausbildung dient dem Zweck, bei der erstgenannten Lösung vorzusehen, dass die Überwurfhülse auf den zylindrischen Mittelabschnitt eben dieses Doppelkonus aufgeschrumpft werden kann. Hierdurch lässt sich eine vollkommen feste und starre Verbindung zwischen Überwurfhülse und Zwischenstück herstellen.

Alternativ gemäß der zweiten Lösung kann vorgesehen sein, dass in dem zylindrischen Mittelabschnitt des Doppelkonus wenigstens eine Nut eingelassen ist, in welche ein Ring aus Silikon gelegt ist, der die Überwurfhülse elastisch festlegt. Die Dimensionen sind so gewählt, dass die Überwurfhülse zwar ortsfest auf dem Zwischenstück gelagert ist, in radialer Richtung jedoch leichte Ausgleichbewegungen oder Mikrobewegungen ausführen kann.

Schließlich wird es bevorzugt, wenn die metallischen Wollen aus Titanfasern bestehen.

Keime oder Schmutzpartikel können beim Einsatz des erfindungsgemäßen Lagers nicht zum Knochenstumpf vordringen sondern werden wirksam abgeblockt. Ein versehentliches Eindringen von beispielsweise Keimen durch Anwendung einer Injektionsnadel ist nicht möglich, da die Überwurfhülse aus einem starren, vorzugsweise metallischen Material besteht. Besonders bevorzugt wird Titan als Werkstoff für beide Hülsen.

Die Erfindung wird anhand der Zeichnungsfiguren beispielhaft näher erläutert. Hierbei zeigt:
- Figur 1: schematisch den Sitz des subkutanen Lagers in einem Oberschenkelstumpf,
- Figur 2: die Überwurfhülse,
- Figur 3: die Klemmhülse,
- Figur 4: die ineinandergefügten vorerwähnten Hülsen,
- Figur 5: das Lager gemäß der alternativen Ausführungsform in einem Oberschenkelstumpf,
- Figur 6: die Überwurfhülse,
- Figur 7: die Klemmhülse,
- Figur 8: die vorerwähnten ineinandergefügten Hülsen.

Nachfolgend bezeichnen gleiche Bezugszeichen gleiche Teile.

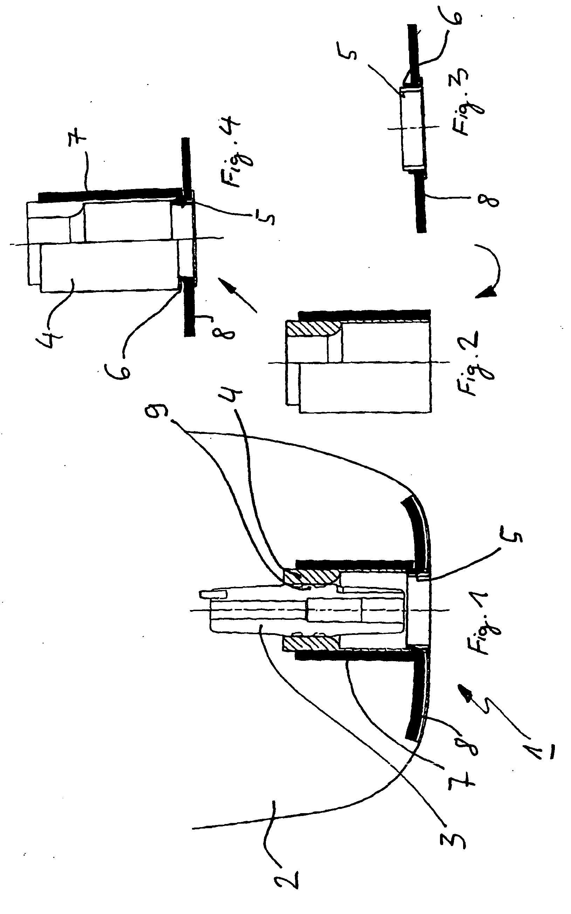

Einen ersten Überblick verschafft Figur 1. Darin ist dargestellt ein Oberschenkelstumpf 2, in welchen das subkutane, intramuskuläre Lager implantiert ist. Das starre transkutane Implantat, das intrakorporal in dem Knochenstumpf verankert ist, ist nicht dargestellt. Ebenfalls nicht dargestellt ist die extrakorporale Kopplungseinrichtung, welche an das Zwischenstück 3 ankoppelbar ist. Das Zwischenstück 3 ist vorliegend als Doppelkonus ausgebildet mit einem zylindrischen Mittelabschnitt 9. An den oberen Konus des Zwischenstückes 3 wird das starre Implantat angekoppelt, wohingegen an den unteren Konus eine extrakorporale Kopplungseinrichtung für ein exoprothetisches Standardteil angekoppelt werden kann.

Auf den Mittelabschnitt 9 des Zwischenstückes 3 ist die Überwurfhülse 4 aufgeschrumpft, so dass das Zwischenstück 3 und die Überwurfhülse 4 eine feste Einheit bilden.

Wie die Figuren 1, 2 und 4 zeigen, ist die Überwurfhülse 4 vorliegend außen mit einer Schicht aus einer metallischen Wolle belegt. Diese Wolle besteht bevorzugt aus Titanfasern. Sie gestattet das Einwachsen benachbarten Gewebes, wodurch eine Keimschranke aufgebaut wird.

Nach extrakorporal, also vorliegend unten, ist die Überwurfhülse 4 offen. In diese Öffnung wird nun eine Klemmhülse 5 (Figur 3) eingeführt. Die Klemmhülse 5 weist außen eine Schicht aus einer metallischen Wolle 6 auf. Beim Hineindrücken der Klemmhülse 5 in die Überwurfhülse 4 kommt es aufgrund der Dimensionen zu einer Kompression der metallischen Wolle 6 im Inneren der Überwurfhülse 4, die ihrerseits zu einem starken Klemmsitz der Hülse 5 in der Überwurfhülse 4 führt.

Wie in Figur 3 gezeigt, ist die metallische Wolle 6 an der Außenwandung der Klemmhülse 5 so fortgeführt, dass sich ein quasi konzentrischer Flansch 8 aus metallischer Wolle ergibt. Dies führt zu einer weiteren Abdichtung an der Stirnseite des Oberschenkelstumpfes 2, wie in Figur 1 gezeigt.

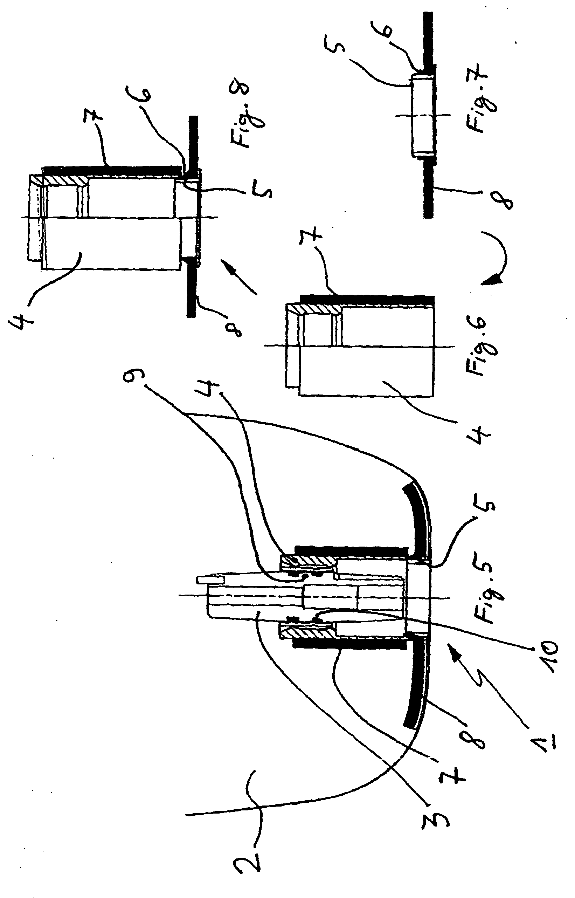

Der Alternativvorschlag gemäß den Figuren 5 bis 8 ist ganz ähnlich aufgebaut. Nachfolgend werden lediglich die konstruktiven Unterschiede kurz erläutert.

Vorliegend ist die Überwurfhülse 4 nicht auf den Mittelabschnitt 9 des Zwischenstückes 3 aufgeschrumpft. Vielmehr ist in den Mittelabschnitt 9 wenigstens eine Nut 10, im dargestellten Ausführungsbeispiel zwei Nuten 10 eingelassen, in welche ein Ring aus Silikon eingelegt ist. Die Dimensionen führen beim Einführen des Zwischenstückes 3 in die Überwurfhülse 4 zu einem ortsfesten Sitz der Hülse 4 auf dem Zwischenstück 3. Aufgrund der Elastizität der Silikonringe aber kann das Lager Mikrobewegungen in radialer Richtung ausführen. Der übrige Aufbau des Lagers entspricht im Wesentlichen jenem, wie er aus den Figuren 1 bis 4 ersichtlich ist. Daher wird auf die entsprechenden Beschreibungsteile verwiesen.

## Patentansprüche

1. Subkutanes, intramuskuläres Lager (1) für ein starres transkutanes Implantat, welches intrakorporal in einem Knochenstumpf verankerbar ist und welches ein Zwischenstück (3) zwischen dem intrakorporal zu verankernden Teil und einer daran ankoppelbaren extrakorporalen Kopplungseinrichtung aufweist,
**gekennzeichnet durch**
eine mit dem Zwischenstück (3) fest verbundene starre Überwurfhülse (4), die in Richtung intrakorporal verschlossen und in Richtung extrakorporal offen ist,
eine Klemmhülse (5), die in die Überwurfhülse (4) in deren offenes Ende setzbar ist, wobei die Klemmhülse (5) von einer metallischen Wolle (6) umgeben ist und die Dimensionen so gewählt sind, dass die Klemmhülse (5) in die Überwurfhülse (4) nur unter Kompression der metallischen Wolle (6) setzbar ist, derart, dass die Kompression für einen dauerhaften Verbleib der Klemmhülse (5) in der Überwurfhülse (4) sorgt.

2. Lager nach Anspruch 1, **dadurch gekennzeichnet, dass** die Überwurfhülse (4) außen mit einer metallischen Wolle (7) belegt ist.

3. Lager nach Anspruch 1 oder 2, **dadurch gekennzeichnet, dass** die metallische Wolle (6), welche die Klemmhülse (5) umgibt, sich distalseitig fortsetzt und einen umlaufenden Flansch (8) bildet.

4. Lager nach einem der Ansprüche 1 bis 3, **dadurch gekennzeichnet, dass** das Zwischenstück (3) ein Doppelkonus mit einem zylindrischen Mittelabschnitt (9) ist.

5. Lager nach einem der Ansprüche 1 bis 4, **dadurch gekennzeichnet, dass** die Überwurfhülse (4) auf den zylindrischen Mittelabschnitt (9) des Doppelkonus aufgeschrumpft ist.

6. Subkutanes, intermuskuläres Lager (1) für ein starres transkutanes Implantat, welches intrakorporal in einem Knochenstumpf (2) verankerbar ist und welches ein Zwischenstück (3) zwischen dem intrakorporal zu verankernden Teil und einer daran ankoppelbaren extrakorporalen Kopplungseinrichtung aufweist,
**gekennzeichnet durch**
eine an dem Zwischenstück (3) elastisch festgelegte starre Überwurfhülse (4), die in Richtung intrakorporal verschlossen und in Richtung extrakorporal offen ist,
eine Klemmhülse (5), die in die Überwurfhülse (4) in deren offenes Ende setzbar ist, wobei die Klemmhülse (5) von einer metallischen Wolle (6) umgeben ist und die Dimensionen so gewählt sind, dass die Klemmhülse (5) in die Überwurfhülse (4) nur unter Kompression der metallischen Wolle (6) setzbar ist, derart, dass die Kompression für einen dauerhaften Verbleib der Klemmhülse (5) in der Überwurfhülse (4) sorgt.

7. Lager nach Anspruch 6, **dadurch gekennzeichnet, dass** die Überwurfhülse (4) außen mit einer metallischen Wolle (7) belegt ist.

8. Lager nach Anspruch 6 oder 7, **dadurch gekennzeichnet, dass** die metallische Wolle (6), welche die Klemmhülse (5) umgibt, sich distalseitig fortsetzt und einen umlaufenden Flansch (8) bildet.

9. Lager nach einem der Ansprüche 6 bis 8, **dadurch gekennzeichnet, dass** das Zwischenstück (3) ein Doppelkonus mit einem zylindrischen Mittelabschnitt (9) ist.

10. Lager nach einem der Ansprüche 6 bis 9, **dadurch gekennzeichnet, dass** in den zylindrischen Mittelabschnitt (9) des Doppelkonus wenigstens eine Nut (10) eingelassen ist, in welche ein Ring aus Silikon gelegt ist, der die Überwurfhülse (4) elastisch festgelegt.

11. Lager nach einem der Ansprüche 1 bis 10, **dadurch gekennzeichnet, dass** die metallischen Wollen (6,7) aus Titanfasern bestehen.

## Claims

1. Subcutaneous, intramuscular bearing (1) for a rigid transcutaneous implant, which can be anchored intracorporeally in a bone stump and which has an intermediate piece (3) between the part to be anchored intracorporeally and an extracorporeal coupling device which can be coupled thereto, **characterised by** a rigid bushing (4) firmly connected to the intermediate piece (3) and which is closed in the intracorporeal direction and open in the extracorporeal direction, a clamping sleeve (5) which can be placed in the bushing (4) in its open end, wherein the clamping sleeve (5) is surrounded by metallic wool (6) and the dimensions are selected so that the clamping sleeve (5) can be placed in the bushing (4) only with compression of the metallic wool (6) such that the compression ensures permanent positioning of the clamping sleeve (5) in the bushing (4).

2. Bearing according to claim 1, **characterised in that** the bushing (4) is covered externally by a metallic wool (7).

3. Bearing according to claim 1 or 2, **characterised in that** the metallic wool (6), which surrounds the clamping sleeve (5), is continued on the distal side and forms an annular flange (8).

4. Bearing according to one of claims 1 to 3, **characterised in that** the intermediate piece (3) is a double cone with a cylindrical central section (9).

5. Bearing according to one of claims 1 to 4, **characterised in that** the bushing (4) is shrunk onto the cylindrical central section (9) of the double cone.

6. Subcutaneous, intermuscular bearing (1) for a rigid transcutaneous implant, which can be anchored intracorporeally in a bone stump (2) and which has an intermediate piece (3) between the part to be anchored intracorporeally and an extracorporeal coupling device which can be coupled thereto, **characterised by** a rigid bushing (4) resiliently fixed to the intermediate piece (3) and which is closed in the intracorporeal direction and open in the extracorporeal direction, a clamping sleeve (5) which can be placed in the bushing (4) in its open end, wherein the clamping sleeve (5) is surrounded by metallic wool (6) and the dimensions are selected so that the clamping sleeve (5) can be placed in the bushing (4) only with compression of the metallic wool (6) such that the compression ensures permanent positioning of the clamping sleeve (5) in the bushing (4).

7. Bearing according to claim 6, **characterised in that** the bushing (4) is covered externally by a metallic wool (7).

8. Bearing according to claim 6 or 7, **characterised in that** the metallic wool (6), which surrounds the clamping sleeve (5), is continued on the distal side and forms an annular flange (8).

9. Bearing according to one of claims 6 to 8, **characterised in that** the intermediate piece (3) is a double cone with a cylindrical central section (9).

10. Bearing according to one of claims 6 to 9, **characterised in that** at least one groove (10), into which a ring made from silicone is placed, which resiliently fixes the bushing (4), is admitted in the cylindrical central section (9) of the double cone.

11. Bearing according to one of claims 1 to 10, **characterised in that** the metallic wools (6, 7) consist of titanium fibres.

## Revendications

1. Logement intramusculaire sous-cutané (1) pour un implant transcutané rigide, qui peut être fixé dans un bout d'os à l'intérieur du corps et qui présente une pièce intermédiaire (3) entre la partie à fixer à l'intérieur du corps et un dispositif d'accouplement extracorporel pouvant lui être raccordé,
**caractérisé par**
une douille d'accouplement (4) rigide connectée fixement à la pièce intermédiaire (3), qui est fermée dans la direction intracorporelle et ouverte dans la direction extracorporelle,
une douille de serrage (5) qui peut être placée dans la douille d'accouplement (4) dans son extrémité ouverte, la douille de serrage (5) étant entourée par une laine métallique (6) et les dimensions étant choisies de telle sorte que la douille de serrage (5) puisse être placée dans la douille d'accouplement (4) uniquement par compression de la laine métallique (6), de telle sorte que la compression assure une fixation durable de la douille de serrage (5) dans la douille d'accouplement (4).

2. Logement selon la revendication 1, **caractérisé en ce que** la douille d'accouplement (4) est garnie à l'extérieur d'une laine métallique (7).

3. Logement selon la revendication 1 ou 2, **caractérisé en ce que** la laine métallique (6), qui entoure la douille de serrage (5), se prolonge du côté distal et forme une bride périphérique (8).

4. Logement selon l'une quelconque des revendications 1 à 3, **caractérisé en ce que** la pièce intermédiaire (3) est un double cône avec une portion centrale cylindrique (9).

5. Logement selon l'une quelconque des revendications 1 à 4, **caractérisé en ce que** la douille d'accouplement (4) est emmanchée par frettage sur la portion centrale cylindrique (9) du double cône.

6. Logement intramusculaire sous-cutané (1) pour un implant transcutané rigide, qui peut être fixé dans un bout d'os (2) à l'intérieur du corps et qui présente une pièce intermédiaire (3) entre la partie à fixer à l'intérieur du corps et un dispositif d'accouplement extracorporel pouvant lui être raccordé,
**caractérisé par**
une douille d'accouplement (4) rigide fixée élastiquement sur la pièce intermédiaire (3), qui est fermée dans la direction intracorporelle et ouverte dans la direction extracorporelle,
une douille de serrage (5) qui peut être placée dans la douille d'accouplement (4) dans son extrémité ouverte, la douille de serrage (5) étant entourée par une laine métallique (6) et les dimensions étant choisies de telle sorte que la douille de serrage (5) puisse être placée dans la douille d'accouplement (4) uniquement par compression de la laine métallique (6), de telle sorte que la compression assure une fixation durable de la douille de serrage (5) dans la douille d'accouplement (4).

7. Logement selon la revendication 6, **caractérisé en ce que** la douille d'accouplement (4) est garnie à l'extérieur d'une laine métallique (7).

8. Logement selon la revendication 6 ou 7, **caractérisé en ce que** la laine métallique (6), qui entoure la douille de serrage (5), se prolonge du côté distal et forme une bride périphérique (8).

9. Logement selon l'une quelconque des revendications 6 à 8, **caractérisé en ce que** la pièce intermédiaire (3) est un double cône avec une portion centrale cylindrique (9).

10. Logement selon l'une quelconque des revendications 6 à 9, **caractérisé en ce qu'**au moins une rainure (10) est pratiquée dans la portion centrale cylindrique (9) du double cône, dans laquelle est placée une bague en silicone qui fixe élastiquement la douille d'accouplement (4).

11. Logement selon l'une quelconque des revendications 1 à 10, **caractérisé en ce que** les laines métalliques (6, 7) se composent de fibres de titane.
